# EUROPÄISCHE PATENTANMELDUNG

(11) **EP 1 857 059 A1**
(43) Veröffentlichungstag der Anmeldung: **21.11.2007**
(21) Anmeldenummer: 07008399.3
(22) Anmeldetag: 25.04.2007
(51) Int. Cl.: A61B 17/22, A61B 17/32, A61B 1/05, A61B 5/00, A61B 17/00, A61B 19/00

(54) **Vorrichtung zur Führung und Leitung eines Instruments in einem Körper**

(30) Priorität: 16.05.2006 DE 102006023108
(71) Anmelder: Dr. Ottow, Manfred, 13465 Berlin (DE)
(72) Erfinder: Ottow, Manfred Dr., 13465 Berlin (DE); Selenz, Hans-Joachim Prof. Dr.-Ing., 31224 Peine (DE); Stark, Michael Prof. Dr. med., 10405 Berlin (DE); Linke, Joachim Dr. med., 13469 Berlin (DE)
(74) Vertreter: Zinken-Sommer, Rainer

(57) **Zusammenfassung**

Die Erfindung betrifft eine Vorrichtung zur Führung und Leitung eines Instruments während des Einführens in einen Körper und des nachfolgenden Navigierens innerhalb des Körpers.

Erfindungsgemäß ist an der Spitze eines Instruments, insbesondere eines minimal-invasiven Instruments, mindestens ein Sensor für Luft- und/oder Körperschall und/oder mindestens ein Sensor für Temperatur und/oder mindestens ein Sensor für Licht, der für Licht von mindestens zwei unterschiedlichen Wellenlängenbereichen empfindlich ist, angeordnet.

Insbesondere Bewegungen von Flüssigkeiten oder Kontraktionen von Muskeln eines Körpers oder Körperteils sind mit Vibrationen oder Schwingungen in Form von Körperschall, d.h. Schwingungen in festem Gewebe, oder Schallwellen, d.h. Schwingungen in Flüssigkeiten oder Gasen, verbunden. Diese Schwingungen werden umso deutlicher spür- bzw. hörbar, je näher man sich einer durchbluteten Blutbahn, einem kontrahierenden Körperteil wie dem Herzen oder einer von Luft durchströmten Trachea nähert. Erfindungsgemäß misst insbesondere ein Mikrofon in Form eines Hydrofons oder ein piezoelektrischer Beschleunigungssensor diese Schwingungen. Die entsprechenden Ausgangssignale werden an eine außerhalb des Körpers befindliche Auswerteeinheit übermittelt und dort einer Bedienperson oder einem Operateur kenntlich gemacht.

## Beschreibung

Die Erfindung betrifft eine Vorrichtung zur Führung und Leitung eines Instruments während des Einführens in einen Körper und des nachfolgenden Navigierens innerhalb des Körpers.

Gattungsgemäße Instrumente werden im Maschinenbau zur Inspektion und Reparatur von Maschinen und Maschinenbauteilen sowie in der Medizin zur Diagnose und Therapie im menschlichen und tierischen Körper verwendet, insbesondere bei der minimal-invasive Chirurgie.

Bei der minimal-invasiven Chirurgie wird durch eine relativ kleine Öffnung mit einer Schnittlänge von insbesondere 0,3 cm bis 2 cm mindestens ein Instrument in das Körperinnere eingebracht. Die Öffnung wird insbesondere durch einen Trokar erzeugt und durch einen Tubus offen gehalten. Ein Operateur kann nach Herausziehen des Trokars aus dem Tubus mit einem Endoskop oder einer Optik durch den Tubus in das Körperinnere schauen oder mit Greif-, Schneide- und anderen Instrumenten im Körperinneren operieren.

Ein Endoskop enthält Lichtleiter für die Beleuchtung und Optik sowie Lichtleiter um das Bild des Eingriffsbereichs nach außen zu übermitteln. Die Optik ist insbesondere in einem Tubus mehr oder weniger weit zurückgezogen, damit sie nicht von den Körperflüssigkeiten benetzt wird und das Bild dadurch verschlechtert wird.

Hierbei werden zwei unterschiedliche Ausführungsformen von Endoskopen verwendet: Endoskope, die aus einem starren Rohr bestehen, sowie flexible Endoskope, die über ein flexibles Rohr verfügen, das individuell in gekrümmte Kanäle eingeführt werden kann.

Derartige Endoskope weisen üblicherweise zwei Kanäle innerhalb des Rohres auf, wobei in einem Kanal Geräte zur Durchführung von Inspektionen und Reparaturen bzw. Diagnose und Therapie eingeführt werden. In einem weiteren Kanal werden insbesondere in dem Körper abgetrennte Teile aus dem Körper entfernt oder Materialien zur Spülung der Optik oder der Geräte eingeführt und abgesaugt.

Außer dem Endoskop werden zusätzlich oder alternativ langstielige Geräte mit typischerweise Haken an den Enden in den Körper eingeführt, um die Position bestimmter Gewebeteile oder Organe zu verändern oder für die eigentliche chirurgische Bearbeitung zu optimieren.

Ebenfalls werden zusätzlich oder alternativ langstielige Schneid- und Verödungsinstrumente eingeführt, die insbesondere mittels Hitze, elektrischem Strom oder Laserstrahlen Gewebe durchtrennen und/oder koagulieren. Diese Instrumente werden von außen mit Manipulatoren bedient.

Im Stand der Technik werden Instrumente in einen menschlichen oder tierischen Körper eingeführt und mittels eines außerhalb des Körpers befindlichen Röntgengerätes oder Tomografen die Führung und Leitung innerhalb des Körpers durchgeführt und überwacht. Wird z.B. über besondere Farb- oder Markierungsstoffe eine Blutbahn für ein Röntgengerät oder einen Tomografen sichtbar gemacht, kann der Bediener oder Operateur des Endoskops sowohl die Blutbahn als auch die Bewegung des Endoskops innerhalb der Blutbahn überprüfen und gegebenenfalls ändern.

Alternativ oder ergänzend beobachtet der Bediener oder Operateur eines Endoskops über an der Spitze des Endoskops befindliche Kameras, Spiegel oder andere optische Übertragungselemente direkt den im Bereich der Spitze des Endoskops befindlichen Raum innerhalb des Körpers. Hierbei ist jedoch insbesondere in durchbluteten Gefäßen aufgrund der nur sehr geringen Sichtweite nur ein sehr begrenzter Raum innerhalb des Körpers sichtbar.

Besonderer Nachteil des Standes der Technik ist jedoch, dass durch die teilweise stark eingeschränkte Führ- und Leitbarkeit eines minimal-invasiven Instruments mittels Röntgengeräten, Tomografen oder optischen Beobachtungsmitteln in einem menschlichen oder tierischen Körper häufig Verletzungen mit zum Teil lebensgefährlichen Folgen entstehen können. So werden insbesondere Blutbahnen durchtrennt, Luftröhren oder Bronchien geschädigt oder irrtümlich statt karzinomem Gewebe gesundes Gewebe entfernt.

Es ist somit Aufgabe der Erfindung, eine Vorrichtung bereitzustellen, mit der eine Erhöhung der Sicherheit und Zuverlässigkeit der Führung und Leitung eines Instruments in einem Körper gewährleistet wird.

Diese Aufgabe wird in Verbindung mit dem Oberbegriff des Anspruches 1 erfindungsgemäß durch die in Anspruch 1 angegebenen Merkmale gelöst.

Die Unteransprüche beinhalten vorteilhafte Ausführungsformen der erfindungsgemäßen Lösung aus Anspruch 1.

Erfindungsgemäß ist an der Spitze eines Instruments, insbesondere eines minimal-invasiven Instruments, mindestens ein Sensor für Luft- und/oder Körperschall und/oder mindestens ein Sensor für Temperatur und/oder mindestens ein Sensor für Licht, der für Licht von mindestens zwei unterschiedlichen Wellenlängenbereichen empfindlich ist, angeordnet.

Insbesondere Bewegungen von Flüssigkeiten oder Kontraktionen von Muskeln eines Körpers oder Körperteils sind mit Vibrationen oder Schwingungen in Form von Körperschall, d.h. Schwingungen in festem Gewebe, oder Schallwellen, d.h. Schwingungen in Flüssigkeiten oder Gasen, verbunden. Diese Schwingungen werden umso deutlicher spür- bzw. hörbar, je näher man sich einer durchbluteten Blutbahn, einem kontrahierenden Körperteil wie dem Herzen oder einer von Luft durchströmten Trachea nähert. Erfindungsgemäß misst insbesondere ein Mikrofon in Form eines Hydrofons oder ein piezoelektrischer Beschleunigungssensor diese Schwingungen. Die entsprechenden Ausgangssignale werden an eine außerhalb des Körpers befindliche Auswerteeinheit übermittelt und dort einer Bedienperson oder einem Operateur kenntlich gemacht.

Überschreiten die ermittelten Schwingungen einen zuvor insbesondere empirisch ermittelten Grenzwert, d.h. nähert sich das Instrument einem gefährdeten Bereich oder Körperteil, erzeugt die Auswerteeinheit ein Warnsignal. Die Bedienperson oder der Operateur kann daraufhin die Position des Instruments erneut überprüfen und gegebenenfalls einen alternativen Weg für die weitere Bewegung der Spitze des Instruments ermitteln, um insbesondere den gefährdeten Bereich oder das Körperteil zu umgehen.

Alternativ oder zusätzlich ermittelt mindestens in Temperatursensor an der Spitze des Instruments die Temperatur des umliegenden Gewebes.

Bekanntlich weisen Organe innerhalb des menschlichen oder tierischen Körpers eine unterschiedliche Temperatur auf, wobei der Temperaturunterschied typischerweise im Bereich von ± 0,01°K liegt. Des Weiteren weisen stark durchblutete Bereiche, wie Entzündungen oder Tumore, eine gegenüber dem umliegenden Gewebe erhöhte Temperatur auf.

Diese Temperaturunterschiede werden von dem mindestens einen Temperatursensor ermittelt. Nähert sich die Spitze des Instruments einem Temperaturunterschied, bedeutet dies somit für die Bedienperson oder den Operateur, dass sich die Spitze des Instruments einem zu umgehenden Organ oder dem Ziel, nämlich der zu bekämpfenden Entzündung oder dem zu bekämpfenden Tumor nähert.

Alternativ oder zusätzlich ermittelt mindestens ein Sensor für Licht, der für Licht von mindestens zwei unterschiedlichen Wellenlängenbereichen empfindlich ist, an der Spitze des Instruments das vom Körper reflektierte oder von speziellen Markerstoffen ausgesandte Licht.

Vorteilhaft wird ein Sensor verwendet, der gleichzeitig für visuelles und gleichzeitig für infrarotes Licht empfindlich ist. Alternativ werden zwei getrennte Sensoren verwendet, wobei der eine für visuelles Licht und der andere für infrarotes Licht empfindlich ist.

In der Auswerteeinheit werden die Bilder beider Wellenlängenbereiche übereinander gelegt, so dass gleichzeitig das visuelle und das infrarote Bild des umliegenden Gewebes beobachtet werden. Wärmeres Gewebe strahlt bekanntlich Licht mit einem höheren infraroten Anteil ab als kühleres Gewebe. Somit werden durch das infrarote Bild Temperaturunterschiede des Körpers im Bereich der Spitze des Endoskops sichtbar. Hierdurch ergibt sich für die Bedienperson oder den Operateur eine weitere Möglichkeit, die Spitze des Instruments in ungefährdete Bereiche oder zum Ziel zu führen.

Ebenso sind anstatt des infraroten Wellenlängenbereichs andere Wellenlängenbereiche verwendbar, die sich aufgrund von unterschiedlichen Eigenschaften unterschiedlichen Gewebes oder unterschiedlicher Flüssigkeiten und Gase vom sichtbaren Bereich unterscheiden.

Die erfindungsgemäßen Sensoren befinden sind vorteilhaft an der Spitze des Instruments, mit dem auch die nachfolgende oder gleichzeitige Diagnose und/oder Therapie durchgeführt wird. Ebenso können sich jedoch die erfindungsgemäßen Sensoren an der Spitze eines Instruments befinden, dass zusätzlich zu dem Instrument, das die Diagnose und/oder Therapie durchführt, in den Körper eingeführt wird. Dieses Instrument überwacht somit die Bewegungen des Instruments für die Diagnose und/oder Therapie.

Der mindestens eine erfindungsgemäße Sensor muss sich nicht bereits bei der ersten Einführung des Instruments in den Körper an der Spitze des Instruments befinden. Er kann vielmehr auch erst zu einem späteren Zeitpunkt, d.h. wenn sich das Instrument bereits innerhalb des Körpers befindet, durch einen entsprechenden Kanal des Instruments an die Spitze des Instruments geschoben werden, wenn die Bedienperson oder der Operateur dies für erforderlich hält.

Die Anwendung der erfindungsgemäßen Vorrichtung beschränkt sich nicht nur auf die medizinische Anwendung am menschlichen oder tierischen Körper. Ebenso ist die erfindungsgemäße Vorrichtung zur Inspektion oder Reparatur von Maschinen oder Maschinenteilen anwendbar, bei denen einzelne Bereiche unterschiedliche schwingungstechnische, thermische oder optische Eigenschaften aufweisen.

Die Erfindung wird nachstehend anhand eines Ausführungsbeispiels näher erläutert.

An der Spitze eines minimal-invasiven Instruments für einen medizinischen Eingriff befinden sich ein piezoelektrischer Beschleunigungsaufnehmer, ein hochgenaues Thermometer und eine Kamera für den visuellen und infraroten Wellenlängenbereich. Die Ausgangssignale werden über eine gemeinsame Leitung oder drei getrennte Leitungen durch einen Kanal des Instruments zu einer Auswerteeinheit außerhalb des Instruments geführt.

Nähert sich der Operateur während des Voranschiebens des Instruments mit der Spitze des Instruments einer gefährdeten Blutbahn, erhöht sich aufgrund der Bewegung des Blutes innerhalb der Blutbahn die von dem piezoelektrischen Beschleunigungsaufnehmer ermittelte Schwingungsamplitude.

Des Weiteren ist in einer Arterie das Blut üblicherweise wärmer als das umliegende Gewebe, so dass das Thermometer die Temperaturerhöhung anzeigt und die Kamera die Temperaturerhöhung abbildet.

Mit Hilfe dieser Daten ermittelt der Operateur oder die Auswerteeinheit den optimalen weiteren Weg des Instruments im Körper, ohne dass besonders gefährdete Bereiche verletzt oder durchtrennt werden.

## Patentansprüche

1. Vorrichtung zur Führung und Leitung eines Instruments während des Einführens in einen Körper und des nachfolgenden Navigierens innerhalb des Körpers, **dadurch gekennzeichnet, dass** an der Spitze des Instruments mindestens ein Sensor für Luft- und/oder Körperschall und/oder mindestens ein Sensor für Temperatur und/oder mindestens ein Sensor für Licht, der für Licht von mindestens zwei unterschiedlichen Wellenlängenbereichen empfindlich ist, angeordnet ist und die entsprechenden Ausgangssignale an eine außerhalb des Körpers befindliche Auswerteeinheit übermittelbar sind.

2. Vorrichtung nach Anspruch 1, **dadurch gekennzeichnet, dass** das Instrument ein minimal-invasives Instrument ist.

3. Vorrichtung nach mindestens einem der Ansprüche 1 bis 2, **dadurch gekennzeichnet, dass** das Instrument ein Endoskop und/oder ein Trokar und/oder ein Tubus ist.

4. Vorrichtung nach mindestens einem der vorigen Ansprüche, **dadurch gekennzeichnet, dass** der mindestens eine Sensor durch einen Kanal des Instruments an die Spitze des Instruments schiebbar ist.

5. Vorrichtung nach mindestens einem der vorigen Ansprüche, **dadurch gekennzeichnet, dass** in dem Instrument mindestens ein Kanal zur Einbringung chirurgischer und/oder therapeutischer Instrumente und/oder zur Ein-und Ausbringung von Spülmittel und/oder abgetrennten Partikeln angebracht ist.

6. Vorrichtung nach mindestens einem der vorigen Ansprüche, **dadurch gekennzeichnet, dass** die Auswerteeinheit bei Überschreiten eines festgelegten Grenzwertes für das entsprechende Ausgangssignal ein Warnsignal abgibt.

7. Vorrichtung nach mindestens einem der vorigen Ansprüche, **dadurch gekennzeichnet, dass** die Auswerteeinheit die Ausgangssignale des mindestens einen Sensors für Luft- oder Körperschall für eine Bedienperson hörbar macht.

8. Vorrichtung nach mindestens einem der vorigen Ansprüche, **dadurch gekennzeichnet, dass** der mindestens eine Sensor für Luftschall ein Mikrofon ist.

9. Vorrichtung nach Anspruch 8, **dadurch gekennzeichnet, dass** das Mikrofon ein Hydrofon ist.

10. Vorrichtung nach mindestens einem der vorigen Ansprüche, **dadurch gekennzeichnet, dass** der mindestens eine Sensor für Körperschall ein Beschleunigungsaufnehmer ist.

11. Vorrichtung nach Anspruch 10, **dadurch gekennzeichnet, dass** der Beschleunigungsaufnehmer ein piezoelektrischer Beschleunigungsaufnehmer ist.

12. Vorrichtung nach mindestens einem der vorigen Ansprüche, **dadurch gekennzeichnet, dass** der mindestens eine Sensor für Temperatur für einen Temperaturunterschied im Bereich von 0,01 °K empfindlich ist.

13. Vorrichtung nach mindestens einem der vorigen Ansprüche, **dadurch gekennzeichnet, dass** der mindestens eine Sensor für Licht im visuellen und im infraroten Wellenlängenbereich empfindlich ist.

14. Vorrichtung nach Anspruch 13, **dadurch gekennzeichnet, dass** die Auswerteeinheit die beiden Bilder des sichtbaren und des infraroten Wellenlängenbereichs übereinander legt.

15. Vorrichtung nach mindestens einem der vorigen Ansprüche, **dadurch gekennzeichnet, dass** der Körper ein menschlicher und/oder tierischer Körper ist.

16. Vorrichtung nach mindestens einem der Ansprüche 1 bis 14, **dadurch gekennzeichnet, dass** der Körper eine Maschine und/oder ein Maschinenteil ist.
